# EUROPÄISCHE PATENTSCHRIFT

(11) **EP 4 190 370 B1**
(45) Veröffentlichungstag und Bekanntmachung des Hinweises auf die Patenterteilung: **25.03.2026**
(21) Anmeldenummer: 22210188.3
(22) Anmeldetag: 29.11.2022
(51) Int. Cl.: A61M 1/00, A47L 9/14, B65F 1/00, B65F 1/06

(54) **VORRICHTUNG MIT EINEM BEHÄLTER UND EINEM ABSAUGBEUTEL FÜR EINE SAUGVORRICHTUNG UND MONTAGEVERFAHREN**
DEVICE COMPRISING A CONTAINER AND A SUCTION BAG FOR A SUCTION DEVICE AND ASSEMBLY METHOD
DISPOSITIF COMPRENANT UN RÉCIPIENT ET UN SAC D'ASPIRATION POUR UN DISPOSITIF D'ASPIRATION ET PROCÉDÉ D'ASSEMBLAGE

(30) Priorität: 02.12.2021 EP 21212099
(43) Veröffentlichungstag der Anmeldung: 07.06.2023
(73) Patentinhaber: BPR Swiss GmbH, 3672 Oberdiessbach (CH)
(72) Erfinder: MAURER, Marc, 3600 Thun (CH); MAURER, Marcel, 2560 Nidau (CH); STRAUCH, Patrick, 3512 Walkringen (CH)
(74) Vertreter: Rutz & Partner

(56) Entgegenhaltungen:
- US-A- 3 704 709
- US-A- 3 845 765
- US-A- 5 118 003
- US-A- 5 914 047

## Beschreibung

Die Erfindung betrifft eine Vorrichtung mit einem Behälter und einem für den einmaligen Gebrauch vorgesehenen Absaugbeutel für eine Saugvorrichtung, die für medizinische oder industrielle Anwendungen vorgesehen ist, sowie ein Verfahren zur Montage des Absaugbeutels im Behälter.

Saugvorrichtungen werden in zahlreichen Bereichen der Industrie und der Medizin eingesetzt. Im Bereich der Dentalmedizin dienen Saugvorrichtungen der Erzeugung eines Unterdrucks an einer Absaugkanüle oder Absaugleitung, durch die Flüssigkeit und Feststoffpartikel, insbesondere Zahnschmelzpartikel, aus dem Mund eines Patienten abgesaugt werden.

Eine Saugvorrichtung dieser Art ist beispielsweise aus der EP3172439B1 bekannt. Diese Saugvorrichtung umfasst ein Pumpensystem, welches über eine Anschlussleitung und eine Medientrennvorrichtung, die einen Behälter umfasst, mit einer Absaugleitung verbunden ist. Die Anschlussleitung und die Absaugleitung sind mit der Abdeckung des Behälters verbunden, in dem die angesaugte Flüssigkeit von der Luft getrennt und gespeichert wird. Die Luft wird über die Anschlussleitung weiter zur Saugvorrichtung gefördert.

Der Behälter umfasst eine Überlaufsicherung, die verhindert, dass die Flüssigkeit bei steigendem Pegel zur Absaugleitung gelangen kann. Sobald der Pegel der Flüssigkeit den maximal zulässigen Wert erreicht hat, wird die Absaugleitung innerhalb des Behälters verschlossen.

Sobald der Behälter gefüllt ist, wird der Verschluss oder die Abdeckung des Behälters entfernt und die Flüssigkeit entsorgt. Der Behälter wird mit entsprechendem Aufwand gereinigt, wobei darauf zu achten ist, dass medizinisches Personal nicht in Kontakt mit der Flüssigkeit gerät.

Bevorzugt wird im Behälter daher ein Absaugbeutel vorgesehen, der mit der angesaugten Flüssigkeit gefüllt und anschliessend verschlossen und entsorgt wird. Vorzugsweise werden auffaltbare oder flexible Absaugbeutel verwendet, die in hoher Anzahl mit minimalem Platzbedarf gelagert werden können. Die Abmessungen des aufgefalteten Absaugbeutels, die aus einer Kunststofffolie gefertigt werden, entsprechen zumindest annähernd den Abmessungen des Behälters.

Die WO200793670A1 offenbart einen Behälter mit einem Absaugbeutel für eine Saugvorrichtung, die für medizinische Einsätze vorgesehen ist. Der Behälter ist mit einer Abdeckung abschliessbar, an der der Absaugbeutel befestigt wird. Als Nachteil solcher Vorrichtungen wird angegeben, dass der Absaugbeutel bei der Verbindung der Abdeckung mit dem Behälter zwischen den oberen Rand des Behälters und die Abdeckung geraten kann, weshalb sich der Absaugbeutel möglicherweise nicht richtig entfalten kann und die Abdeckung den Behälter möglicherweise nicht dicht abschliesst. Weiter wird darauf hingewiesen, dass eine fehlerhafte Montage des Absaugbeutels insbesondere bei medizinischen Notfällen leicht auftreten kann. Zur Vermeidung dieses Problems werden Befestigungsmittel vorgesehen, welche den Absaugbeutel im gefalteten Zustand halten, bis dieser in den Behälter eingesetzt ist. Nach dem Einsetzen des Absaugbeutels muss hingegen sichergestellt werden, dass das Befestigungsmittel die Auffaltung des Absaugbeutels nicht länger behindert.

Typischerweise ist der Absaugbeutel mit der Abdeckung fest verbunden und wird zusammen mit der Abdeckung entsorgt, weshalb ein hoher Materialverbrauch und ein hoher Kostenaufwand zur Herstellung des Absaugbeutels resultieren. Weiter resultiert ein hoher Aufwand zur Entsorgung der Absaugbeutel, da die massive Abdeckung aus Plastik zu entsorgen ist.

Sofern der Absaugbeutel hingegen vom Anwender mit der Abdeckung verbunden wird, ist dies nur mit Geschick möglich. Der Saugbeutel muss präzise an ein Halteelement der Abdeckung angepasst sein und gegebenenfalls anhand eines Montagemittels, wie eines elastischen Rings, montiert werden.

Falls der gefüllte Absaugbeutel nicht zusammen mit der Abdeckung entsorgt wird, muss er nach dem Füllvorgang vorsichtig von der Abdeckung gelöst werden, um zu verhindern, dass Flüssigkeit freigesetzt wird. Dabei besteht die Gefahr, dass sich der gefüllte Absaugbeutel selbsttätig von der Abdeckung löst und die Flüssigkeit freigesetzt wird.

Der Absaugbeutel mit dem zusätzlichen Befestigungsmittel ist daher relativ aufwendig gestaltet und nur mit Geschick montierbar und demontierbar.

Zu beachten ist, dass bei der Montage dieser bekannten Absaugbeutel die Abdeckung manuell oder durch Saugdruck an den Behälter angepresst wird, weshalb zwischen dem Behälter und der Abdeckung normalerweise keine absolut dichte Verbindung resultiert.

Die WO200793670A1 offenbart ferner, dass im Raum zwischen dem Behälter und dem Absaugbeutel ein Unterdruck erzeugt wird, damit sich der Absaugbeutel entfalten kann und durch den Unterdruck, der innerhalb des Absaugbeutels vorherrscht, zusammengezogen wird.

Die US3845765A offenbart weitere Vorrichtungen für eine Saugvorrichtung, die einen Behälter aufweisen, in dem ein Absaugbeutel angeordnet wird und der mittels mit einer Abdeckung abschliessbar ist. Bei einer ersten Ausgestaltung der Vorrichtung wird der Rand des Absaugbeutels zwischen dem oberen Rand des Behälters und der Abdeckung über einen Dichtungsring nach aussen geführt, was mit verschiedenen Nachteilen verbunden ist. Sofern der Rand des Absaugbeutels über dem Dichtungsring gefaltet ist, was beim Einsetzen leicht passieren kann, entstehen Spalten oder Mikrokanäle, durch die hindurch abgesaugte Flüssigkeit nach aussen gelangen kann. Bei der Montage des Absaugbeutels ist daher darauf zu achten, dass über dem Dichtungsring keine Überlappungen resultieren. Besonders unvorteilhaft ist auch die Handhabung des Absaugbeutels nach dessen Füllung. Bei der Entfernung der Abdeckung wird der Rand des gefüllten Absaugbeutels nicht mehr gehalten, weshalb dieser durch das Gewicht der angesaugten Flüssigkeit in den Behälter zurückgezogen wird und abgesaugte Flüssigkeit über den Rand des Absaugbeutels in den Behälter hinein oder nach aussen gelangen kann. Nachteilig ist ferner das Erscheinungsbild der Vorrichtung mit dem nach aussen geführten Rand des Absaugbeutels, der einen unvorteilhaften ästhetischen Eindruck hinterlässt und mit dem das medizinische Personal nicht in Kontakt gelangen möchte.

In einer weiteren Ausgestaltung der Vorrichtung der US3845765A wird vorgeschlagen, den Absaugbeutel an einem Gestell zu befestigen, das nach der Montage des Absaugbeutels in den Behälter der Vorrichtung hineingestellt wird. Der Rand des Absaugbeutels wird zwischen einem ersten Ring und einem zweiten Ring des Gestells hindurch geführt. Indem der zweite Ring gegen den ersten Ring geführt wird, wird der Rand des Beutels festgeklemmt und fixiert. Bei dieser Ausgestaltung der Vorrichtung kann der Absaugbeutel einschliesslich des Beutelrands vollständig innerhalb des Behälters angeordnet werden. Zur Entfernung des gefüllten Absaugbeutels wird die Abdeckung vom Behälter entfernt und das Gestell an einem Bügel ergriffen und aus dem Behälter angehoben. In der Folge werden die beiden Ringe voneinander gelöst und der Absaugbeutel entfernt. Auf diese Ausgestaltung der Vorrichtung ist mit zahlreichen Nachteilen verbunden. Es ist ein Gestell mit zwei Klemmringen erforderlich, das einen Herstellungsaufwand und entsprechende Kosten verursacht und mit dem der Absaugbeutel mit Geschick zu verbinden ist, indem der Rand des Absaugbeutels zwischen den beiden Ringen hindurchgeführt und fixiert wird. Besonders schwierig ist die Handhabung wiederum nach der Füllung des Beutels, da beim Lösen der beiden Klemmringe der Beutel unter Einwirkung des Gewichts der angesaugten Flüssigkeit leicht entgleiten kann und angesaugte Flüssigkeit freigesetzt wird. Weiter ist zu beachten, dass das Gestell einen erheblichen Wartungsaufwand verursacht, da es nach der Benutzung zu reinigen ist. Sofern das Gestell feingliedrige Elemente umfasst, besteht ferner die Gefahr, dass diese deformiert werden und Korrekturmassnahmen erforderlich sind. Weiterhin nimmt das Gestell innerhalb des Behälters Raum in Anspruch.

Die US5914047A offenbart eine weitere Vorrichtung für eine Saugvorrichtung, die einen Behälter aufweist, in dem ein Absaugbeutel angeordnet wird. Bei dieser Vorrichtung weist der Behälter ein Halsstück auf, durch das das mit der Öffnung versehene Endstück des Absaugbeutels hindurch und über das Halsstück nach aussen gefaltet wird. Das Halsstück ist mittels einer Schutzabdeckung abdeckbar, mittels der auch der Absaugbeutel fixiert wird. Durch die Schutzabdeckung erfolgt hingegen kein dichter Abschluss des Absaugbeutels, weshalb anderweitig dafür zu sorgen ist, dass die abgesaugte Flüssigkeit nicht austreten kann. Diese Vorrichtung ist bedingt somit eine entsprechende Ausgestaltung, die nur mit erheblichem Aufwand realisierbar ist.

Die US5118003A offenbart eine weitere Vorrichtung für eine Saugvorrichtung, die einen Behälter aufweist, in dem ein Absaugbeutel angeordnet wird und der mittels mit einer Abdeckung abschliessbar ist. Die Abdeckung, der Absaugbeutel und ein Montagering bilden zusammen eine Einwegeinheit, die nach Gebrauch bzw. nach dem Füllen des Absaugbeutels entsorgt wird. Der Montagering, der auf den Rand des Behälters aufsetzbar ist und die Abdeckung weisen zueinander korrespondierende Verzahnungen auf, zwischen denen der Rand des Absaugbeutels nach aussen geführt wird, bevor die Verzahnungen ineinander verpresst werden, um die Einwegeinheit zu bilden. Für diesen Pressvorgang bzw. für die Fertigstellung der Einwegeinheit ist eine Pressvorrichtung vorgesehen, mittels der der Rand des Absaugbeutels in einem ersten Schritt fixiert wird, bevor die Verzahnungen der Abdeckung und des Montagerings ineinander gepresst werden. Auch diese Vorrichtung hat zahlreiche Nachteile. Die Abdeckung, der Montagering und der Beutel, die eine Einwegeinheit bilden sind nach Gebrauch zu entsorgen, weshalb viel Verbrauchsmaterial mit hohen Kosten bereitgestellt und entsorgt werden muss. Zur Fertigung der Einwegeinheit ist ein Werkzeug erforderlich, welches Kosten verursacht und Raum in Anspruch nimmt. Der Rand des Absaugbeutels wird auch bei dieser Vorrichtung nach aussen geführt, weshalb auch bei dieser Vorrichtung die oben mit Bezug zur US5914047A beschriebenen technischen und ästhetischen Nachteile resultieren.

Die US3704709A offenbart einen Absaugbeutel, der nach Gebrauch verschlossen werden kann.

Der vorliegenden Erfindung liegt daher die Aufgabe zugrunde, eine verbesserte Vorrichtung mit einem Behälter und einem für den einmaligen Gebrauch vorgesehenen Absaugbeutel für eine Saugvorrichtung zu schaffen, die bei medizinischen und industriellen Anwendungen einsetzbar ist. Weiterhin ist ein verbessertes Montagefahren anzugeben, mittels dessen der Absaugbeutel vorteilhaft im Behälter der Vorrichtung montierbar ist.

Der Saugbeutel soll einfach ausgestaltet und mittels des Montageverfahrens in einfacher Weise im Behälter montierbar und nach dem Füllen mit abgesagter Flüssigkeit wieder demontierbar sein.

Insbesondere soll es nicht erforderlich sein, den Absaugbeutel mit Geschick zu montieren und mit Geschick zu demontieren, um zu verhindern, dass nach der Montage undichte Stellen resultieren und sich der Absaugbeutel bei der Demontage möglicherweise selbsttätig löst und die abgesaugte Flüssigkeit freigesetzt wird. Fehlmanipulationen sollen bei der Montage und Demontage des Absaugbeutels somit zuverlässig vermieden werden.

Die Vorrichtung soll einfach ausgestaltet sein. Eine Hilfsvorrichtung, wie ein Gestell, das mit dem Absaugbeutel zu verbinden und in den Behälter einzusetzen ist, soll vermieden werden.

Weiterhin soll vermieden werden, dass ein Teil des Absaugbeutels zur Aussenseite des Behälters geführt wird und störend in Erscheinung tritt.

Für den Betrieb der Vorrichtung soll nebst dem Absaugbeutel kein weiteres Verbrauchsmaterial oder Einwegmaterial bereitgestellt und entsorgt werden müssen.

Weiterhin sollen Werkzeuge oder Apparate vermieden werden, mittels denen der Absaugbeutel beispielsweise mit der Abdeckung und/oder einem Montagering verbunden wird.

Auf besondere Montagemittel, die den Absaugbeutel gefaltet halten, bevor er montiert wird, und die zu lösen sind, bevor der Absaugbeutel gefüllt wird, soll verzichtet werden können. Dadurch soll auch vermieden werden, dass der Absaugbeutel teilweise gefaltet werden muss bevor er montiert wird und daher entsprechend viel Raum in Anspruch nimmt.

Weiterhin soll darauf verzichtet werden können, innerhalb des Behälters zwischen dem Absaugbeutel und der Wand des Behälters einen Unterdruck zu erzeugen.

Die erfindungsgemässe Vorrichtung soll auch bei hohen Ansaugleistungen von beispielsweise 100 l/min - 300 l/min einwandfrei funktionieren. Insbesondere soll es auch bei hohen Ansaugleistungen nicht notwendig sein, einen Unterdruck zwischen dem Absaugbeutel und dem Behälter vorzusehen.

Diese Aufgabe wird mit einer Vorrichtung mit einem Behälter und einem Absaugbeutel gemäss Anspruch 1, einem Absaugbeutel gemäss Anspruch 10 und einem Montageverfahren gemäss Anspruch 11 gelöst. Vorteilhafte Ausgestaltungen der Erfindung sind in weiteren Ansprüchen angegeben.

Die Vorrichtung, die für eine Saugvorrichtung im Bereich medizinischer oder industrieller Anwendungen vorgesehen ist, umfasst
- einen vorzugsweise aus Plastik gefertigten Behälter, der eine Behälterwand aufweist, die eine Innenseite und eine Aussenseite aufweist, die einen Behälterraum umschliesst, und an die an der Unterseite ein Behälterboden und an der Oberseite eine Behälteröffnung anschliesst,
- eine Abdeckung zum Schliessen der Behälteröffnung,
- einen Montagering, und
- einen für den einmaligen Gebrauch vorgesehenen Absaugbeutel, der an einem Ende eine Beutelöffnung aufweist und der innerhalb des Behälterraums vom Montagering gehalten, umschlossen und in ein einseitig geschlossenes Unterteil und in ein mit der Beutelöffnung versehenes Oberteil aufteilt ist, welches Oberteil über den Montagering und einen Teil des Unterteils zurückgefaltet ist, sodass der Montagering eine Aufnahmeöffnung des Absaugbeutels begrenzt.

Erfindungsgemässe ist vorgesehen, dass an der Innenseite der Behälterwand wenigstens ein Stützelement angeformt ist, auf das der Montagering derart abgestützt ist, dass das Oberteil des Absaugbeutels innerhalb des Behälterraums zwischen dem Montagering und der Behälterwand gegen den Behälterboden verläuft.

Auf Vorrichtungsteile, wie ein Gestell, die mit dem Absaugbeutel zu verbinden sind und die zusammen mit dem montierten Absaugbeutel in den Behälter einzusetzen sind, kann somit verzichtet werden. Die Vorrichtung kann ohne Gestell kostengünstig hergestellt werden. Der Raum innerhalb des Behälters kann optimal genutzt werden.

Der Absaugbeutel wird vom Montagering sicher gehalten, sodass die Abdeckung problemlos entfernt werden kann, nachdem der Absaugbeutel gefüllt wurde.

Der Rand des Absaugbeutels ragt auch nicht zur Aussenseite des Behälters hinaus, sondern wird innerhalb des Behälters zurück gegen den Behälterboden geführt.

Die Montage des Absaugbeutels erfolgt in einfacher Weise, ohne dass Werkzeuge oder Apparate benötigt werden.

Gemäss dem erfindungsgemässen Verfahren zur Montage des Absaugbeutels in der Vorrichtung ist vorgesehen
- dass in einem ersten Montageschritt der Montagering über den Absaugbeutel geführt wird, sodass der Montagering den Absaugbeutel umschliesst und in das einseitig geschlossene Unterteil und in das mit der Beutelöffnung versehene Oberteil aufteilt,
- dass in einem zweiten Montageschritt das Oberteil radial nach aussen über den Montagering und einen Teil des Unterteils zurückgefaltet wird, sodass der Montagering eine Aufnahmeöffnung des Absaugbeutels begrenzt,
   dass in einem dritten Montageschritt der Absaugbeutel in den Behälterraum des geöffneten Behälters eingeführt und der Montagering auf das wenigstens eine Stützelement abgestützt wird, und
- dass in einem vierten Montageschritt die Abdeckung oder ein mit der Abdeckung verbundener Anschlussring auf den Behälter aufgesetzt wird.

Sofern angrenzend an die Beutelöffnung eine Zugleine vorgesehen ist, mittels der der Absaugbeutel abschliessbar ist, so kann die Zugleine nach dem zweiten Montageschritt in einem optionalen Zwischenschritt leicht angezogen werden. Durch diesen optionalen Zwischenschritt wird der Montagering vom Beutelrand weiter umschlossen und festgehalten. Ein abschliessbarer Absaugbeutel weist in Verbindung mit der erfindungsgemässen Vorrichtung daher besondere Vorteile nicht nur hinsichtlich der Entsorgung, sondern auch hinsichtlich der Montage auf.

Falls dies nicht bereits geschehen ist, wird die Vorrichtung durch eine an die Abdeckung angeschlossene Anschlussleitung mit der Saugvorrichtung verbunden und die Saugvorrichtung in Betrieb gesetzt. Über eine Absaugleitung, die mit der Abdeckung verbunden ist, kann in der Folge Flüssigkeit in den Absaugbeutel transferiert werden, wobei angesaugte Luft und Flüssigkeit innerhalb des Behälters voneinander getrennt werden. Die angesaugte Luft wird über die Anschlussleitung weiter zur Saugvorrichtung geführt, während die Flüssigkeit im Behälter gespeichert wird.

Nach Abschluss des Saugvorganges wird die Abdeckung oder der mit der Abdeckung verbundene Anschlussring vom Behälter gelöst und der Montagering erfasst und mit dem Absaugbeutel aus dem Behälter entfernt. Nach dem Lösen der Abdeckung ist der Montagering frei zugänglich, sodass er bequem erfasst und angehoben werden kann.

Der Absaugbeutel umfasst vorzugsweise eine Zugleine, die nach der Entfernung des mit Flüssigkeit gefüllten Absaugbeutels aus dem Behälter betätigt wird, um die Beutelöffnung dicht abzuschliessen. Die Zugleine ist beispielsweise in Ösen oder in einem schlauchförmigen Abschlussstück des Absaugbeutels gehalten, das gegebenenfalls die Beutelöffnung begrenzt.

Der verschlossene Absaugbeutel kann nun nach Entfernen des Montagerings oder zusammen mit dem Montagering sicher entsorgt werden. Sofern der Montagering entfernt wird, kann er in der Folge mit einem neuen Absaugbeutel verbunden werden.

Die Sicherheit in der Handhabung des Absaugbeutels kann weiter erhöht werden, indem Flüssigkeit absorbierendes Material im Absaugbeutel vorgesehen wird.

Der Vorgang zur Montage und Demontage des Absaugbeutels kann mit einfachen Massnahmen durchgeführt werden, ohne dass sich der Absaugbeutel selbsttätig lösen und Flüssigkeit aus dem Beutel austreten kann.

Zur Montage des Absaugbeutels muss dieser nicht mit der Abdeckung verbunden werden. Eine Verbindung mit der Abdeckung verursacht Schwierigkeiten, weil der Absaugbeutel dicht abschliessend mit der Abdeckung zu verbinden und dabei zu beachten ist, dass sich der Absaugbeutel nicht wieder selbsttätig löst. Stattdessen wird das mit der Beutelöffnung versehene Endstück des Absaugbeutels durch den Montagering hindurch geführt und radial nach allen Seiten über den Montagering hinweg und zurückgeführt. Bei diesem Vorgang ist lediglich der Montagering und nicht die gesamte Abdeckung zu manipulieren. Da das Endstück des Absaugbeutels in einfacher Weise radial nach aussen um den Montagering herumgeführt wird resultiert praktisch automatisch eine zuverlässige Verbindung zwischen dem Montagering und dem Absaugbeutel.

Der mit dem Montagering versehene Absaugbeutel kann nun in einfacher Weise in den Behälter eingesetzt werden, ohne dass die Abdeckung, die normalerweise mit Leitungen verbunden ist, störend in Erscheinung tritt. Erst nachdem der Absaugbeutel bequem in den Behälter eingesetzt und der Montagering auf das wenigstens eine Stützelement aufgesetzt wurde, wird der Behälter mit der Abdeckung verschlossen. Dabei werden die eingangs beschriebenen Probleme aus dem Stand der Technik vermieden, wonach der mit der Abdeckung verbundene Absaugbeutel zwischen der Abdeckung und dem Behälter festgeklemmt werden kann. Stattdessen wurde der Absaugbeutel vollständig in den Behälter eingesetzt, sodass die Abdeckung ohne jegliche Schwierigkeiten aufgesetzt werden kann und ein dichter Abschluss zwischen dem Behälter und der Abdeckung oder zwischen dem Behälter und dem mit der Abdeckung verschraubten Anschlussring resultiert.

Nach Abschluss des Saugvorgangs kann die Abdeckung wieder bequem und ohne besondere Vorsicht entfernt werden, da der Absaugbeutel nicht mit der Abdeckung verbunden ist. Es besteht somit auch keine Gefahr, dass sich der Absaugbeutel während der Entnahme durch mögliche Krafteinwirkungen von der Abdeckung löst. Stattdessen kann der Montagering erfasst und mit dem Absaugbeutel aus dem geöffneten Behälter entfernt werden. Das über den Montagering geführte Oberteil des Absaugbeutels kann nun wieder zurückgeführt und durch Betätigung der Zugleine verschlossen werden. Bei diesen Vorgang wird der Montagering wieder freigelegt, sodass er bequem entfernt und wieder verwendet werden kann. Da das Oberteil des Beutels um den Montagering herumgeführt wurde, wurde auch eine Kontaktierung des Montagerings mit der angesaugten Flüssigkeit vermieden. Der Montagering ist daher nicht kontaminiert.

Die beschriebenen Montageschritte können alle vom Anwender in einfacher Weise durchgeführt werden. Die Montageschritte zur Verbindung des Montagerings mit dem Absaugbeutel können jedoch auch auf Seiten des Herstellers durchgeführt werden, sodass dem Anwender Absaugbeutel zur Verfügung gestellt werden, die bereits einen Montagering aufweisen und direkt in den Behälter eingesetzt werden können. In diesem Fall ist nur ein dünner Montagering und nicht, wie bei den eingangs beschriebenen Lösungen, eine gesamte Abdeckung mit dem Absaugbeutel zu entsorgen.

Der Montagering ist vorzugsweise elastisch deformierbar und/oder aus einem hartelastischen oder weichelastischen Material gefertigt. Die Elastizität des Montagerings erlaubt es, diesen in einfacher Weise zu montieren und/oder den Beutelrand vorzugsweise dicht abschliessend gegen die Innenseite der Beutelwand und/oder das wenigstens eine Stützelement zu drücken.

Das wenigstens eine Stützelement ist vorzugsweise als umlaufende Stufe oder Schulter an der Innenseite der Behälterwand angeformt oder als umlaufende Nut in die Behälterwand eingesenkt, wobei die Unterseite der Nut vorzugsweise eine Schulter bildet. Das wenigstens eine Stützelement kann daher vorteilhaft einstückig an der Innenseite der Behälterwand angeformt oder ausgeformt sein. Der Montagering kann beispielsweise auf die Schulter aufgelegt oder formschlüssig in die umlaufende Nut eingefügt werden. Am Montagering wird vorzugsweise wenigstens ein vorstehendes Greifelement vorgesehen, welches in den Behälterraum hineinragt und ergriffen werden kann, um den Montagering zu lösen und/oder anzuheben.

Das Stützelement kann in einer nicht beanspruchten Ausführungsform ein Ringelement sein, das in eine umlaufende Nut in der Behälterwand eingesetzt wird. Stützelemente können auch einstückig als Ausformungen, gegebenenfalls Auswölbungen, an der Gehäusewand realisiert werden.

Das Stützelement oder die Stützelemente können daher in einfacher Weise realisiert werden, ohne dass ein Gestell oder dergleichen benötigt wird.

Der Montagering kann mit einem Handgriff auf das wenigstens eine Stützelement, gegebenenfalls die Schulter aufgesetzt und/oder formschlüssig mit der Innenseite der Behälterwand verbunden, gegebenenfalls in eine umlaufende Nut eingesetzt werden, in der der elastische Montagering gehalten ist und erst durch Krafteinwirkung wieder gelöst werden kann. Der Montagering ist daher vorzugsweise elastisch ausgebildet und aus elastischem Material gefertigt. Besonders vorteilhaft ist die Anordnung des Montagerings auf der Schulter, durch die der Montagering fest gehalten wird und mittels der Abdeckung oder des Anschlussrings gegebenenfalls komprimiert und vorzugsweise dicht abschliessend gegen die Schulter gedrückt werden kann. Dadurch wird vermieden, dass angesaugte Flüssigkeit in den Behälter gelangen und sich am Behälterboden ansammeln kann.

In einer weiteren vorzugsweisen Ausgestaltung ist vorgesehen, dass die Abdeckung oder der mit der Abdeckung verbundene Anschlussring einen Flanschring aufweist, mittels dessen ein Dichtungsring dicht abschliessend gegen eine ringförmig verlaufende Oberkante des Behälters gedrückt wird.

Zur Montage der Abdeckung ist die Innenwand des Behälters zwischen der Behälteröffnung und dem wenigstens einen Stützelement vorzugsweise mit einem Innengewinde versehen, auf das die Abdeckung oder der mit der Abdeckung verschraubte Anschlussring aufgeschraubt werden kann.

Der in einer vorzugsweisen Ausgestaltung vorgesehene Anschlussring weist vorzugsweise auf einer Seite ein mit dem Behälter verschraubbares erstes Gewindeteil und auf der anderen Seite ein mit der Abdeckung verschraubbares zweites Gewindeteil sowie einen aussen liegenden ringförmigen Flanschring auf, durch den der Dichtungsring dicht abschliessend gegen die Oberkante des Behälters gedrückt wird.

Durch die durch Gewindeelemente realisierte Verbindung der Abdeckung gegebenenfalls des Anschlussrings mit der Abdeckung mit dem Behälter resultiert eine stabile und bereits relativ dicht abgeschlossene Verbindung zwischen dem Behälter und der Abdeckung.

Der Behälter wird zudem mittels des Montagerings, der gegen die ringförmig verlaufende Schulter innerhalb des Behälters gedrückt wird, und/oder mittels des Dichtungsrings, der gegen die ringförmig verlaufende Oberkante des Behälters gedrückt wird, dicht abgeschlossen.

Es hat sich gezeigt, dass der Absaugbeutel bei diesem dichtem Abschluss des Behälters und auch bei hohen Saugleistungen nicht gegen die Abdeckung gezogen wird und sich frei entfalten kann. Es ist daher nicht notwendig, zwischen der Aussenseite des Absaugbeutels und der Innenseite des Behälters einen Unterdruck zu erzeugen. Ein solcher Unterdruck wird mittels einer Hilfsleitung, die mit der Anschlussleitung oder direkt mit der Saugvorrichtung verbunden ist, nur optional vorgesehen.

Nachfolgend wird die Erfindung anhand von Zeichnungen näher erläutert. Dabei zeigt:
- Fig. 1a: eine erfindungsgemässe Vorrichtung 1 mit einem Behälter 2 und einem darin angeordneten Absaugbeutel 3 (schematisch gezeigt) sowie mit einer Abdeckung 6, die durch einen Anschlussring 5 mit dem Behälter 2, durch eine Anschlussleitung 82 mit einer Saugvorrichtung 8 und durch eine Absaugleitung 81 mit einem Saugkopf 811 verbunden ist;
- Fig. 1b: die Vorrichtung 1 von Fig. 1 mit dem Absaugbeutel 3, dem Behälter 2 und dem Anschlussring 5 in einem Längsschnitt mit Blick auf eine Überlaufsicherung 91, die innerhalb des Behälters 2 den Pegelstand der Flüssigkeit misst;
- Fig. 2: einen Teil der Vorrichtung 1 von Fig. 1 mit einem Teil des Absaugbeutels 3, der innerhalb des Behälters 2 von einem Montagering 4 gehalten ist;
- Fig. 3: die Abdeckung 6, den Anschlussring 5 und einen Teil des Behälters 2 von Fig. 2 voneinander getrennt in Schnittdarstellung;
- Fig. 4a: den Absaugbeutel 3 von Fig. 1 in einer vorzugsweisen Ausgestaltung bei einem ersten Schritt des Montageverfahrens, bei dem dem Absaugbeutel 3 der Montagering 4 übergestreift wird, der den Absaugbeutel 3 in einen abgeschlossenen unteren Teil 31 und einen mit der Beutelöffnung 30 versehene oberen Teil 32 unterteilt; und
- Fig. 4b: den Absaugbeutel 3 von Fig. 4a bei einem zweiten Schritt des Montageverfahrens, bei dem der obere Teil 32 des Absaugbeutels 3 über den Montagering 4 nach unten geführt wird, wonach der Absaugbeutel 3 am Montagering 4 erfasst und in den Behälter 2 eingesetzt werden kann.

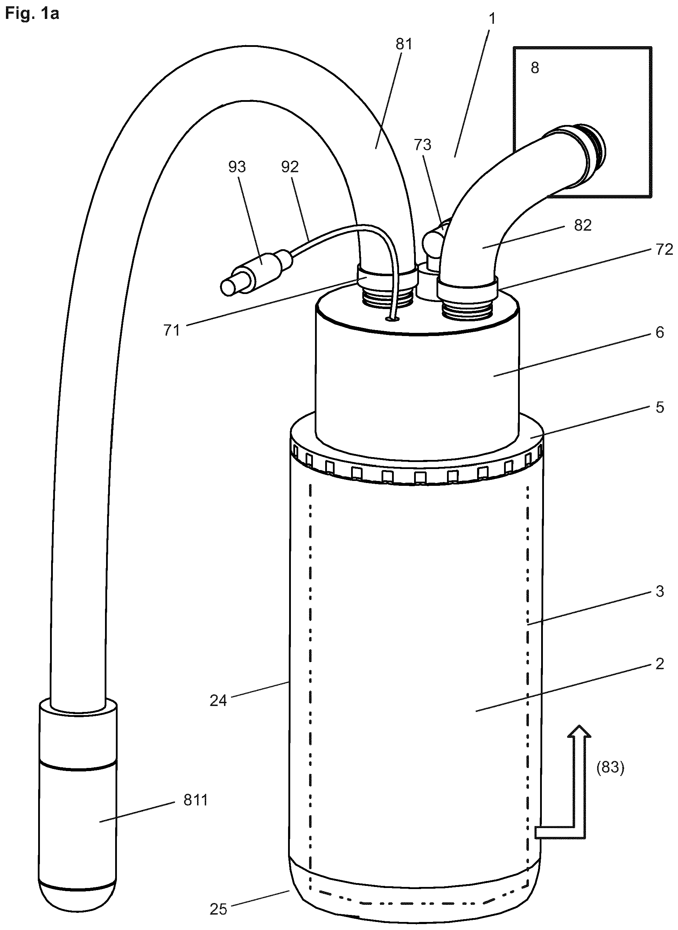

Fig. 1a zeigt eine erfindungsgemässe Vorrichtung 1 mit einem Behälter 2 und einem darin angeordneten Absaugbeutel 3 (strichpunktiert dargestellt).

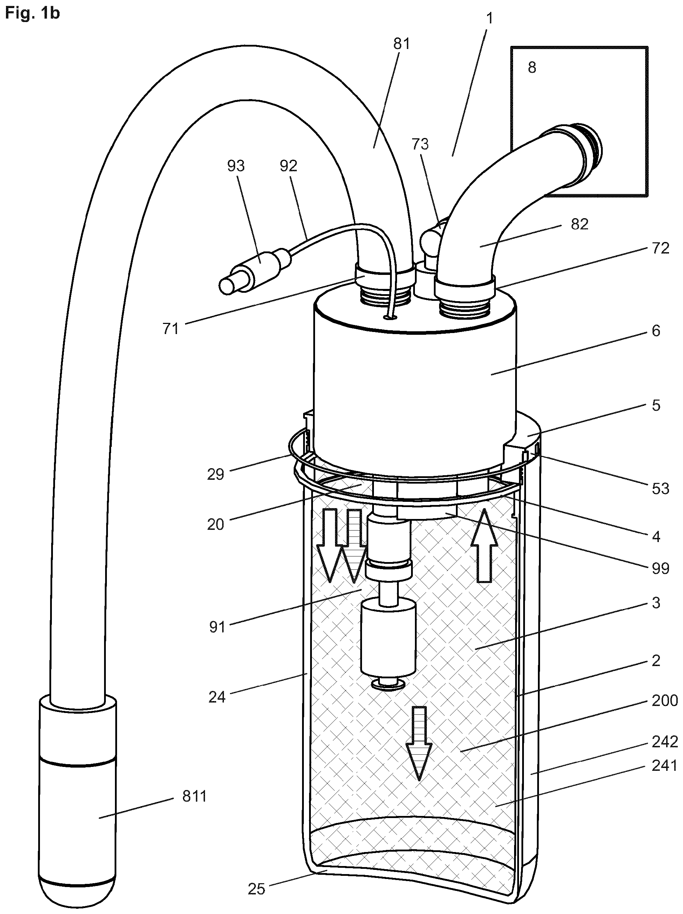

Der Behälter 2 weist eine Behälterwand 24 auf, die eine Innenseite 241 und eine Aussenseite 242 aufweist, die einen Behälterraum 200 umschliesst, und an die an der Unterseite ein Behälterboden 25 und an der Oberseite eine Behälteröffnung 20 anschliesst (siehe Fig. 1b).

Ferner ist eine Abdeckung 6 vorgesehen, die optional durch einen Anschlussring 5 mit dem Behälter 2 verbunden ist. Alternativ kann die Abdeckung 6 auch direkt auf den Behälter 2 aufgesetzt werden. Dies ist beispielsweise möglich, indem der Anschlussring 5 einstückig an der Abdeckung 6 angeformt und nicht durch ein Gewinde mit der Abdeckung 6 verbunden wird. Durch die Wahl eines entsprechenden Anschlussrings 5 können nach Wunsch unterschiedliche Abdeckungen 6 auf den Behälter 2 aufgesetzt werden.

Der Behälter 2 und die Abdeckung sind vorzugsweise aus Kunststoff gefertigt. Der Anschlussring 5 ist vorzugsweise aus Metall gefertigt.

Die Abdeckung 6 ist durch eine Anschlussleitung 82 mit einer Saugvorrichtung 8 und durch eine Absaugleitung 81 mit einem Saugkopf 811 verbunden. Die Anschlussleitung 82 und die Absaugleitung 81 sind mit Anschlusselementen 71, 72 verbunden, die in Gewindebohrungen der Abdeckung 6 angeschraubt sind.

Mit der Abdeckung 6 ist ein Halteelement 73 verbunden, mittels dessen die Vorrichtung 1 beispielsweise mit der Saugvorrichtung 8 verbunden werden kann.

Weiterhin ist eine optionale Hilfsleitung 83 gezeigt, die direkt oder indirekt mit der Saugvorrichtung 8 verbunden wird und über die ein Unterdruck innerhalb des Behälters 2 zwischen der Aussenseite des Absaugbeutels 3 und der Innenwand des Behälters 2 erzeugt werden kann.

Durch die erfindungsgemässe Abdichtung des Behälters 2 kann auf diese Hilfsleitung 83 jedoch verzichtet werden.

Aus der Abdeckung 6 ist zudem ein Messkabel 92 herausgeführt, dass innerhalb des Behälters mit einer Pegelmessvorrichtung oder Überlaufsicherung und ausserhalb des Behälters 2 mit einem Anschlussstecker 93 verbunden ist. Das Messkabel 92 wird mit einer Steuereinheit verbunden, welche die Saugvorrichtung 8 steuert und bei Feststellung eines Überlaufs im Behälter 2 ein optisches oder akustisches Warnsignal generiert oder die Saugvorrichtung 8 abschaltet.

Fig. 1b zeigt die Vorrichtung 1 von Fig. 1 mit dem Absaugbeutel 3, dem Behälter 2 und dem Anschlussring 5 in einem Längsschnitt mit Blick auf die Überlaufsicherung 91, die im Innenraum bzw. Behälterraum 200 des Behälters 2 angeordnet ist und den Pegelstand der Flüssigkeit misst.

Der Anschlussring 5 weist einen Aussenflansch 53 auf, durch den ein Dichtungsring 29 dicht abschliessend mit einer Oberkante des Behälters 2 verbunden wird. Nach dem Aufsetzen der Abdeckung 6 oder der Abdeckung mit dem Anschlussrings 5 ist der Behälter 2 daher dicht abgeschlossen.

Der Behälter 2 weist nahe an der Behälteröffnung 20 ein Innengewinde 21 und unterhalb des Innengewindes 21 eine Stützelement 22 in der Form einer umlaufenden und vorzugsweise in sich geschlossenen Schulter auf. Auf das Stützelement 22 ist ein Montagering 4 abgestützt, von dem der schraffiert gezeigte Absaugbeutel 3 gehalten ist.

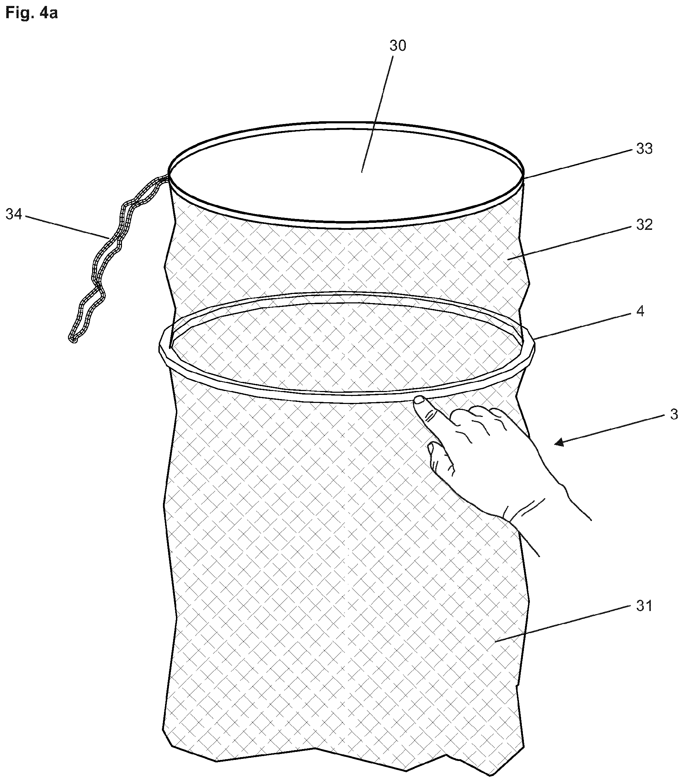

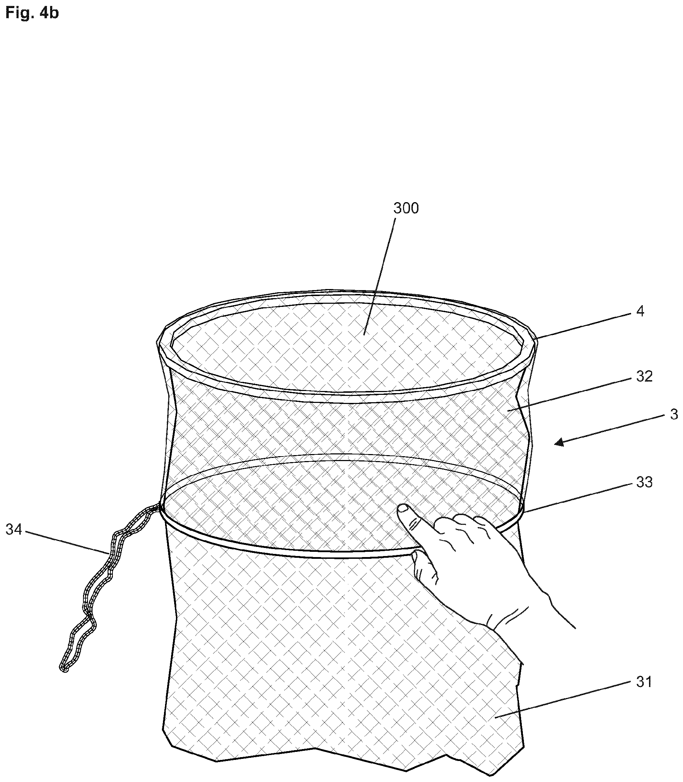

Der Absaugbeutel 3 und die Schritte zur Montage des Absaugbeutels 3 im Behälter 2 sind in Fig. 4a und Fig. 4b illustriert.

Die für den einmaligen Gebrauch vorgesehenen Absaugbeutel 3 sind aus einer Plastikfolie gefertigt, an der Unterseite verschlossen und an der Oberseite mit einer Beutelöffnung 30 versehen. Vorzugsweise anschliessend an die Beutelöffnung 30 ist wenigstens ein Haltemittel 33, wie Schlaufen, Ösen, Schlauchelemente, Rohrelemente oder ein Saum, vorgesehen, von dem ein Zugmittel 34 gehalten ist, mittels dessen die Beutelöffnung 30 abschliessbar ist. Bevorzugt wird ein Saum 33 vorgesehen, der gebildet wird, indem angrenzend an die Beutelöffnung 30 ein Endstück des Absaugbeutels 3 nach aussen gefaltet und mit der Plastikfolie verschweisst wird.

Absaugbeutel 3 werden vorzugsweise in einer Verpackung geliefert, in der eine hohe Anzahl von Absaugbeuteln 3 zugefaltet flächig aufeinander liegen.

Fig. 4a zeigt, dass in einem ersten Montageschritt der Montagering 4 über den Absaugbeutel 3 geführt wurde, sodass der Montagering 4 den Absaugbeutel 3 umschliesst und in ein einseitig geschlossenes Unterteil 31 und in ein mit der Beutelöffnung 30 versehenes Oberteil 32 aufteilt. Zur Vorbereitung dieses Montageschritts wird der Absaugbeutel 3 vorzugsweise aufgefaltet, so dass er beispielsweise eine zylindrische oder schlauchförmige Form annimmt. Der Montagering 4 kann weit nach oben geführt werden, so dass das Oberteil 32 vorzugsweise wesentlich kürzer ist als dargestellt.

Fig. 4b zeigt, dass in einem zweiten Montageschritt das Oberteil 32 radial nach aussen über den Montagering 4 und einen Teil des Unterteils 31 zurückgefaltet wird, sodass der Montagering 4 eine Aufnahmeöffnung 300 des Absaugbeutels 3 begrenzt.

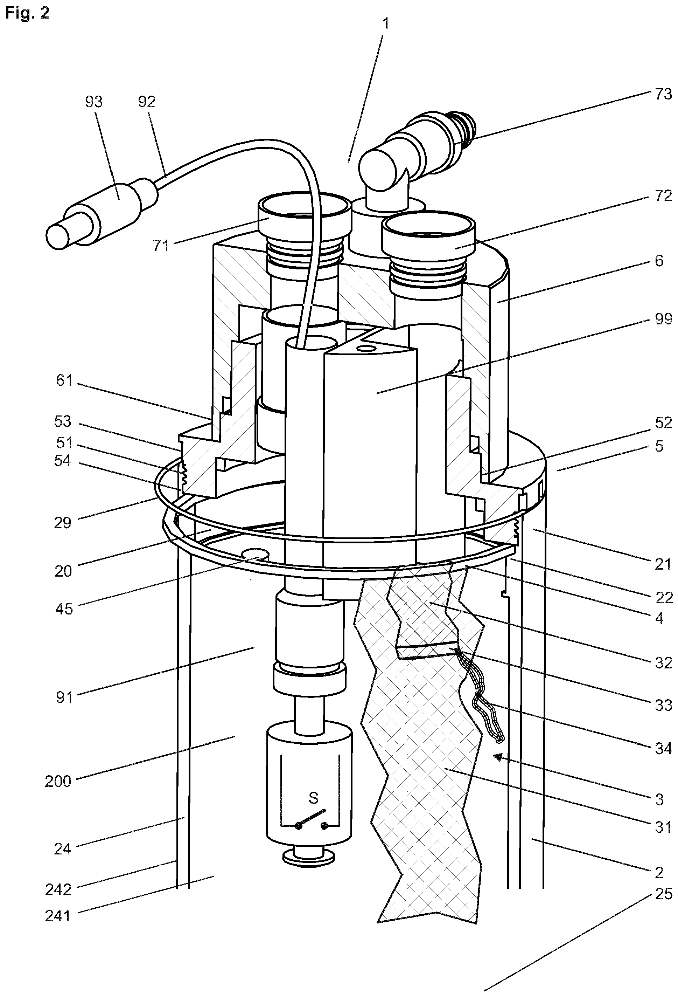

In einem dritten Montageschritt wird der mit dem Montagering 4 verbundene Absaugbeutel 3 in den Behälterraum 200 des geöffneten Behälters 2 eingeführt und der Montagering 4 auf das wenigstens eine Stützelement 22 abgestützt, wie dies in Fig. 2 gezeigt ist.

In einem vierten Montageschritt die Abdeckung 6 bzw. der mit der Abdeckung 6 verbundene Anschlussring 5 auf den Behälter 2 aufgesetzt.

Alle Montageschritte können bequem ausgeführt werden, ohne den Behälter 2 oder die Abdeckung 6 zu manipulieren.

Fig. 2 zeigt, dass der Absaugbeutel 3 am Montagering 4 aufgehängt ist und dass das um den Montagering 4 herum geführte Oberteil 32 des Absaugbeutels 3 mit der Zugleine 34 nach unten hängt bzw. dem Behälterboden 25 zugewandt ist. Dabei liegt nicht der Montagering 4, sondern das Oberteil 32 des Absaugbeutels 3 am schulterförmigen Stützelement 22 und an der Unterseite des Anschlussrings 5 an. Der Montagering 4 ist daher isoliert und kann von der angesaugten Flüssigkeit nicht kontaminiert werden.

In vorzugsweisen Ausgestaltungen der Erfindung wird der Montagering 4 formschlüssig mit der Innenseite der Behälterwand 24 verbunden, gegebenenfalls zwischen obere und untere Stützelemente oder in eine umlaufende Nut, gegebenenfalls Ringnut, eingefügt.

Der Montagering 4 weist vorzugsweise wenigstens ein Greifelement 45 auf, mittels dessen der Montagering 4 ergriffen, gegebenenfalls aus einer formschlüssigen Verbindung mit der Innenseite der Behälterwand 24 gelöst, und angehoben werden kann. Das Greifelement 45 oder die Greifelemente 45 können vorteilhaft einstückig am Montagering 4 angeformt sein oder auch Klammern oder dergleichen sein, die den Montagering 4 umschliessen oder durchstossen.

Das Oberteil 32 des Absaugbeutels 3 verläuft über den Montagering 4, gegebenenfalls über das wenigstens eine Greifelement 45, und weiter innerhalb des Behälterraums 200 zwischen dem Montagering 4 und der Behälterwand 24 nach unten gegen den Behälterboden 25.

Vorzugsweise wird das Oberteil 32 des Absaugbeutels 3 vom Montagering 4 elastisch und dicht abschliessend gegen das Stützelement 22 und/oder gegen die Innenseite 241 der Behälterwand 24 gedrückt. Der Absaugbeutel 3 ist daher vollständig innerhalb des Behälterraums 200 gehalten.

Die Länge des Oberteils 32 des Absaugbeutels 3, welche über den Montagering 4 geführt wird, kann kurz gehalten werden, sodass sie den Montagering 4 und das Unterteil 31 des Absaugbeutels 3 nur wenig, beispielsweise einen bis mehrere Millimeter oder Zentimeter, überlappt.

Besonders vorteilhaft ist die Anordnung des Zugmittels oder der Zugleine 34 am Oberteil 32 des Absaugbeutels 3. Nachdem das Oberteil 32 des Absaugbeutels 3 über den Montagering 4 geführt wurde, kann das Zugmittel 34 in einem optionalen Zwischenschritt des Verfahrens leicht angezogen werden, um den Absaugbeutel 3 am Montagering 4 zu fixieren.

In der Folge kann der Montagering 4 mit dem Absaugbeutel 3 besonders einfach auf das wenigstens eine Stützelement 22 aufgesetzt werden, dass sich der Absaugbeutel 3 nicht selbsttätig vom Montagering 4 lösen kann.

Sofern der Montagering 4 aus weichelastischem Material gefertigt ist, wird er durch die Abdeckung 6 bzw. den Anschlussring 5 vorzugsweise leicht komprimiert, so dass auch der Montagering 4 einen Dichtungsring bildet. Auf den Dichtungsring 29 kann daher verzichtet werden, wenn mit dem Montagering 4 eine genügende Abdichtung des Behälters 2 erzielt wird.

Die Abdeckung 6 ist dicht abgeschlossen mit dem Anschlussring 5 verbunden, der mit dem Behälter 2 verschraubt ist und mit dem Flanschring 53 den Dichtungsring 29 an die Oberkante des Behälters 2 andrückt. Nach dem Aufsetzen der Abdeckung 6 und des optional vorgesehenen Anschlussrings 5 ist der Behälter 2 dicht abgeschlossen.

Fig. 2 zeigt, dass das an die Behälteröffnung 20 anschliessende Innengewinde 21 mit einem ersten Gewindeteil 51 an der Unterseite des Anschlussrings 5 verschraubt ist. Das untere Endstück 54 des ersten Gewindeteils 51 liegt vorzugsweise am Montagering 4 an. Das zweite Gewindeteil 52 an der Oberseite des Anschlussrings 5 ist mit einem Innengewinde eines Anschlussflanschs 61 der Abdeckung 6 verschraubt.

Nach der Montage des Absaugbeutels 3 kann die Absaugvorrichtung in Betrieb genommen werden. Durch die Saugvorrichtung 8 wird über die Anschlussleitung 82 im Behälter 2 ein Unterdruck erzeugt, der durch die Absaugleitung 81 auf den Saugkopf 811 übertragen wird. Bei einem medizinischen Einsatz, z.B. in einer Zahnarztpraxis, werden über den Saugkopf 811 Luft und Flüssigkeit des Patienten angesaugt, wie dies in Fig. 1b mit einem weissen Pfeil und einem schraffierten Pfeil symbolisiert ist.

Die Flüssigkeit (schraffierter Pfeil) wird im Behälter 2 gespeichert, während Luft (weisser Pfeil) um ein Abscheideelement 99 herum und über die Anschlussleitung 82 zur Saugvorrichtung 8 geführt wird.

Fig. 2 zeigt weiterhin, dass die Überlaufsicherung 91 vorzugsweise einen Schwimmer und einen Sensor oder einen Schalter S aufweist, welcher beim Überlauf des Behälters 2 durch die Verschiebung des Schwimmers aktiviert oder betätigt wird. Die Betätigung des Schalters oder Sensors wird von einer Steuereinheit erfasst, welche die Saugvorrichtung 8 entsprechend steuert.

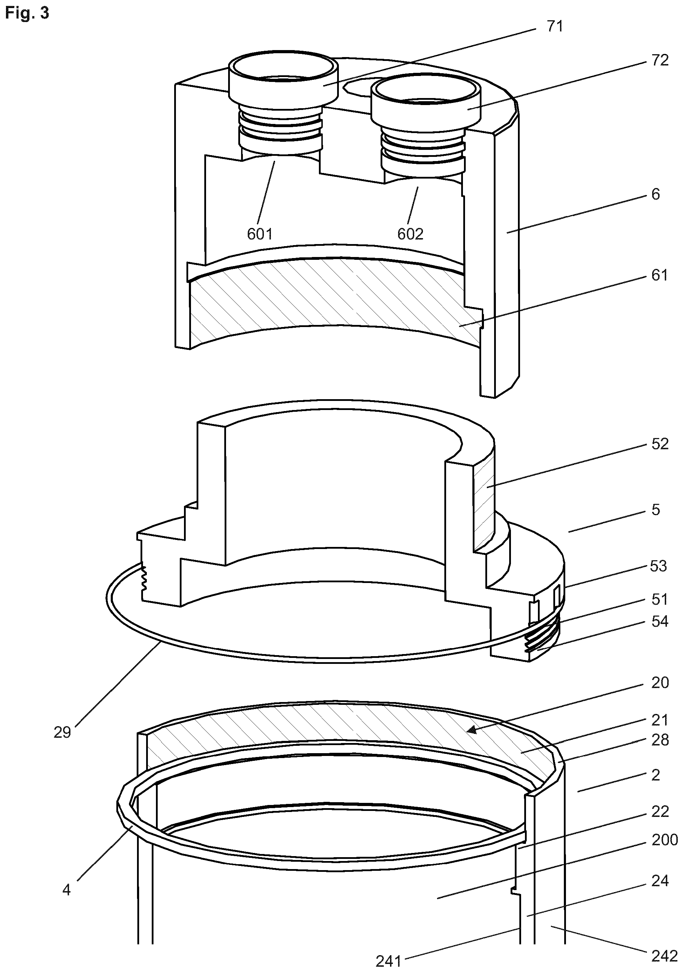

Fig. 3 zeigt die Abdeckung 6 mit den beiden Abschlusselementen 71, 72, die in Gewindebohrungen 601, 602 der Abdeckung 6 eingesetzt sind, den Anschlussring 5 mit dem Dichtungsring 29 und einen Teil des Behälters 2 mit dem Montagering 4 von Fig. 2 voneinander getrennt und in Schnittdarstellung.

Der Montagering 4 ist auf das schulterförmige Stützelement 22 aufgesetzt, das in den Behälterraum 200 hineinragt. Zwischen der Behälteröffnung 20 und den Stützelement 22 ist das schraffiert gezeigte Innengewinde vorgesehen. In das Innengewinde 21 des Behälters 2 wird das mit einem Aussengewinde versehene erste Gewindeteil 51 des Anschlussrings 5 eingeschraubt. Das zweite Gewindeteil 52 des Anschlussrings 5 weist ein schraffiert gezeigtes Aussengewinde auf, welches zu einem schraffiert gezeigten Innengewinde 61 der Abdeckung 6 korrespondiert. Der Dichtungsring 29 liegt unterhalb des Aussenflanschs 53 des Anschlussrings 5 und wird von diesem gegen eine Oberkante 28 des Behälters 2 gedrückt, sobald der Anschlussrings 5 mit dem Behälter 2 verschraubt wird. Bei diesem Vorgang wird die Unterseite 54 des ersten Gewindeteils 51 des Anschlussrings 5 vorzugsweise so weit gegen den Montagering 4 gedreht, dass dieser fixiert und gegebenenfalls komprimiert wird.

Fig. 4a zeigt den Absaugbeutel 3 von Fig. 1 in einer vorzugsweisen Ausgestaltung bei einem ersten Schritt des oben beschriebenen Montageverfahrens.

Fig. 4b zeigt den Absaugbeutel 3 von Fig. 4a bei einem zweiten Schritt des Montageverfahrens.

### Bezugszeichenliste

- 1: Vorrichtung
- 2: Behälter
- 20: Behälteröffnung
- 200: Behälterraum
- 21: Innengewinde
- 22: Stützelement
- 24: Behälterwand
- 241: Innenseite der Behälterwand
- 242: Aussenseite der Behälterwand
- 25: Behälterboden
- 28: Oberkante
- 29: Dichtungsring
- 3: Absaugbeutel
- 30: Beutelöffnung
- 300: Aufnahmeöffnung
- 31: abgeschlossener unterer Teil des Absaugbeutels 3
- 32: obere Teil des Absaugbeutels 3
- 33: Abschlussstück
- 34: Zugleine
- 4: Montagering
- 45: Greifelement
- 5: Anschlussring
- 51: erstes Gewindeteil
- 52: zweites Gewindeteil
- 53: Flanschring
- 54: unteres Endstück 54 des ersten Gewindeteils 51
- 6: Abdeckung
- 601: Gewindebohrung
- 602: Gewindebohrung
- 61: Innengewinde 61 der Abdeckung 6
- 71: Anschlusselement
- 72: Anschlusselementen
- 73: Halteelement
- 8: Saugvorrichtung
- 81: Absaugleitung
- 811: Saugkopf
- 82: Anschlussleitung
- 83: Hilfsleitung
- 91: Überlaufsicherung
- 92: Messkabel
- 93: Anschlussstecker
- 99: Abscheideelement

## Patentansprüche

1. Vorrichtung (1) für eine Saugvorrichtung (8)
mit einem Behälter (2), der eine Behälterwand (24) aufweist, die eine Innenseite (241) und eine Aussenseite (242) aufweist, die einen Behälterraum (200) umschliesst, und an die an der Unterseite ein Behälterboden (25) und an der Oberseite eine Behälteröffnung (20) anschliesst,
mit einer Abdeckung (6) zum Schliessen der Behälteröffnung (20),
mit einem Montagering (4), und
mit einem für den einmaligen Gebrauch vorgesehenen Absaugbeutel (3), der an einem Ende eine Beutelöffnung (30) aufweist und der innerhalb des Behälterraums (200) vom Montagering (4) gehalten, umschlossen und in ein einseitig geschlossenes Unterteil (31) und in ein mit der Beutelöffnung (30) versehenes Oberteil (32) aufteilt ist, welches Oberteil (32) über den Montagering (4) und einen Teil des Unterteils (31) zurückgefaltet ist, sodass der Montagering (4) eine Aufnahmeöffnung (300) des Absaugbeutels (3) begrenzt, **dadurch gekennzeichnet,**
**dass** an der Innenseite (241) der Behälterwand (24) wenigstens ein Stützelement (22) angeformt ist, auf das der Montagering (4) derart abgestützt ist, dass das Oberteil (32) des Absaugbeutels (3) innerhalb des Behälterraums (200) zwischen dem Montagering (4) und der Behälterwand (24) gegen den Behälterboden (25) verläuft.

2. Vorrichtung (1) nach Anspruch 1, **dadurch gekennzeichnet, dass** der Montagering (4) elastisch deformierbar ist und dass der Montagering (4) aus einem hartelastischen oder weichelastischen Material gefertigt ist, so dass das Oberteil (32) des Absaugbeutels (3) zwischen dem Montagering (4) und der Behälterwand (24) vorzugsweise einklemmbar ist.

3. Vorrichtung (1) nach Anspruch 1 oder 2, **dadurch gekennzeichnet,**
a) **dass** das wenigstens eine Stützelement (22) als umlaufende Stufe oder Schulter an der Innenseite der Behälterwand (24) angeformt ist; oder
b) **dass** das wenigstens eine Stützelement (22) eine umlaufende Nut ist, die in der Behälterwand (24) vorgesehen ist und deren Unterseite vorzugsweise eine Schulter bildet; oder
c) **dass** das wenigstens eine Stützelement (22) ein Ringelement ist, das in einer Nut der Behälterwand (24) gehalten ist; oder
d) **dass** Stützelemente (22) als Ausformungen in der Behälterwand (24) vorgesehen sind.

4. Vorrichtung (1) nach einem der Ansprüche 1 - 3, **dadurch gekennzeichnet,**
a) **dass** der Montagering (4) formschlüssig oder kraftschlüssig mit der Innenseite der Behälterwand (24) verbunden oder verbindbar und vorzugsweise mit wenigstens einem Greifelement (45) versehen ist; oder
b) **dass** der Montagering (4) durch die Abdeckung (6) oder durch einen mit der Abdeckung (6) verbundenen Anschlussring (5) in Position gehalten oder vorzugsweise dicht abschliessend gegen das wenigstens eine Stützelement (22) gedrückt wird.

5. Vorrichtung (1) nach einem der Ansprüche 1 - 4, **dadurch gekennzeichnet, dass** die Abdeckung (6) oder der mit der Abdeckung (6) verbundene Anschlussring (5) einen Flanschring (53) aufweist, mittels dessen ein Dichtungsring (29) dicht abschliessend gegen eine ringförmig verlaufende Oberkante oder Stirnseite (28) der Behälterwand (24) gedrückt wird.

6. Vorrichtung (1) nach einem der Ansprüche 1 - 5, **dadurch gekennzeichnet, dass** der Absaugbeutel (3) eine Zugleine (34) umfasst, durch deren Betätigung der Absaugbeutel (3) oder die Beutelöffnung (30) abschliessbar ist, wobei die Zugleine (34) vorzugsweise in Ösen oder in einem schlauchförmigen Abschlussstück (33) des Absaugbeutels (3), das die Beutelöffnung (30) begrenzt, gehalten ist.

7. Vorrichtung (1) nach einem der Ansprüche 1 - 6, **dadurch gekennzeichnet, dass** zwischen der Behälteröffnung (20) und dem wenigstens einen Stützelement (22) ein Innengewinde (21) vorgesehen ist, auf das die Abdeckung (6) oder der mit der Abdeckung (6) verschraubte Anschlussring (5) aufgeschraubt ist.

8. Vorrichtung (1) nach Anspruch 7, **dadurch gekennzeichnet, dass** der Anschlussring (5) auf einer Seite ein mit dem Behälter (5) verschraubtes erstes Gewindeteil (51) und auf der anderen Seite ein mit der Abdeckung (6) verschraubtes zweites Gewindeteil (52) und einen aussen liegenden ringförmigen Flanschring (53) aufweist, durch den der Dichtungsring (29) dicht abschliessend gegen die Oberkante (28) des Behälters (2) gedrückt wird.

9. Vorrichtung (1) nach einem der Ansprüche 1 - 8, **dadurch gekennzeichnet, dass** die Abdeckung (6) durch Anschlusselemente (71, 72) mit einer Anschlussleitung (82), die an die Saugvorrichtung (8) angeschlossen ist, und mit einer Absaugleitung (81) verbunden ist.

10. Nicht-chirurgisches, nicht-therapeutisches Verfahren zur Montage eines für den einmaligen Gebrauch vorgesehenen Absaugbeutels (3), der an einem Ende eine Beutelöffnung (30) aufweist, in einem Behälter (2) einer Vorrichtung (1) nach einem der Ansprüche 1 - 9, welcher Behälter (2) einen Behälterraum (200) mit wenigstens einem darin vorgesehenen Stützelement (22), auf das ein Montagering (4) abstützbar ist, und mit einer daran anschliessenden Behälteröffnung (20) aufweist, die mit einer Abdeckung (6) abschliessbar ist, **dadurch gekennzeichnet,**
**dass** in einem ersten Montageschritt der Montagering (4) über den Absaugbeutel (3) geführt wird, sodass der Montagering (4) den Absaugbeutel (3) umschliesst und in ein einseitig geschlossenes Unterteil (31) und in ein mit der Beutelöffnung (30) versehenes Oberteil (32) aufteilt,
**dass** in einem zweiten Montageschritt das Oberteil (32) radial nach aussen über den Montagering (4) und einen Teil des Unterteils (31) zurückgefaltet wird, sodass der Montagering (4) eine Aufnahmeöffnung (300) des Absaugbeutels (3) begrenzt,
**dass** in einem dritten Montageschritt der Absaugbeutel (3) in den Behälterraum (200) des geöffneten Behälters (2) eingeführt und der Montagering (4) auf das wenigstens eine Stützelement (22) abgestützt wird, und
**dass** in einem vierten Montageschritt die Abdeckung (6) oder ein mit der Abdeckung (6) verbundener Anschlussring (5) auf den Behälter (2) aufgesetzt wird.

11. Verfahren nach Anspruch 10, **dadurch gekennzeichnet, dass** der Montagering (4) durch die Abdeckung (6) oder durch den Anschlussring (5), der mit der Abdeckung (6) verbunden ist oder verbunden wird, dicht abschliessend gegen das wenigstens eine Stützelement (22) gepresst wird.

12. Verfahren nach Anspruch 10 oder 11, **dadurch gekennzeichnet, dass** die Vorrichtung (1) durch eine an die Abdeckung (6) angeschlossene Anschlussleitung (82) mit einer Saugvorrichtung (8) verbunden wird, dass die Saugvorrichtung (8) in Betrieb gesetzt wird und dass über eine Absaugleitung (81), die mit der Abdeckung (6) verbunden ist, Flüssigkeit in den Absaugbeutel (3) transferiert wird, wonach die Abdeckung (6) oder der mit der Abdeckung (6) verbundene Anschlussring (5) vom Behälter gelöst und der Montagering (4) erfasst und mit dem Absaugbeutel (3) aus dem Behälter (2) entfernt wird.

13. Verfahren nach Anspruch 12, **dadurch gekennzeichnet, dass** der Absaugbeutel (3) eine Zugleine (34) umfasst, die nach der Entfernung des Absaugbeutels (3) aus dem Behälter (2) betätigt wird, um die Beutelöffnung (30) abzuschliessen.

14. Verfahren nach Anspruch 12 oder 13, **dadurch gekennzeichnet, dass** der Montagering (4) vom gefüllten Absaugbeutel (3) entfernt und mit einem neuen Absaugbeutel (3) verbunden und der gefüllte Absaugbeutel (3) entsorgt wird oder dass der Montagering (4) mit dem gefüllten Absaugbeutel (3) entsorgt wird.

## Claims

1. Device (1) for a suction device (8)
with a container (2) that has a container wall (24), which has an inner side (241) and an outer side (242), which encloses a container space (200) and which is adjoned at the lower side by a container base (25) and at the upper side by a container opening (20),
with a cover (6) for closing the container opening (20),
with a mounting ring (4), and
with a suction bag (3) intended for single use, which has a bag opening (30) at one end and is held and enclosed within the container space (200) by the mounting ring (4) and is divided into a lower part (31) closed on one side and an upper part (32) provided with the bag opening (30), which upper part (32) is folded back over the mounting ring (4) and a portion of the lower part (31) so that the mounting ring (4) delimits a receiving opening (300) of the suction bag (3), **characterized in,**
**that** at least one support element (22) is formed on the inner side (241) of the container wall (24), on which the mounting ring (4) is supported in such a way that the upper part (32) of the suction bag (3) runs within the container space (200) between the mounting ring (4) and the container wall (24) against the container base (25).

2. Device (1) according to claim 1, **characterized in, that** the mounting ring (4) is elastically deformable and that the mounting ring (4) is made of a hard elastic or soft elastic material, so that the upper part (32) of the suction bag (3) can preferably be clamped between the mounting ring (4) and the container wall (24).

3. Device (1) according to claim 1 or 2, **characterized in,**
a) **that** at least one support element (22) is formed as a circumferential step or shoulder on the inner side of the container wall (24); or
b) **that** the at least one support element (22) is a circumferential groove provided in the container wall (24) and whose lower side preferably forms a shoulder; or
c) **that** the at least one support element (22) is a ring element held in a groove in the container wall (24); or
d) **that** support elements (22) are provided as formations in the container wall (24) what the pelvic site have I have.

4. Device (1) according to one of the claims 1 - 3, **characterized in,**
a) **that** the mounting ring (4) is connected or connectable to the inner side of the container wall (24) in a form-fitting or force-fitting manner and is preferably provided with at least one gripping element (45); or
b) **that** the mounting ring (4) is held in position by the cover (6) or by a connection ring (5) connected to the cover (6) or, preferably, pressed tightly against the at least one support element (22).

5. Device (1) according to one of the claims 1 - 4, **characterized in, that** the cover (6) or the connection ring (5) connected to the cover (6) has a flange ring (53) by means of which a sealing ring (29) is pressed tightly against a ring-shaped upper edge or end face (28) of the container wall (24).

6. Device (1) according to one of the claims 1 - 5, **characterized in, that** the suction bag (3) comprises a pull cord (34) which, when operated, closes the suction bag (3) or the bag opening (30), wherein the pull cord (34) is preferably held in eyelets or in a tubular terminal piece (33) of the suction bag (3) which delimits the bag opening (30).

7. Device (1) according to one of the claims 1 - 6, **characterized in, that** an internal thread (21) is provided between the container opening (20) and the at least one support element (22), onto which the cover (6) or the connection ring (5) screwed to the cover (6) is screwed.

8. Device (1) according to claim 7, **characterized in, that** the connection ring (5) has a first threaded part (51) screwed to the container (5) and, on the other side, a second threaded part (52) screwed to the cover (6) and an outer annular flange ring (53), by means of which the sealing ring (29) is pressed tightly against the upper edge (28) of the container (2).

9. Device (1) according to one of the claims 1 - 8, **characterized in, that** the cover (6) is connected by connecting elements (71, 72) to a connection line (82) which is connected to the suction device (8) and to a suction line (81).

10. Non-surgical, non-therapeutic method for mounting a single-use suction bag (3), which has a bag opening (30) at one end, in a container (2) of a device (1) according to one of claims 1 - 9, which container (2) has a container space (200) with at least one support element (22) provided therein, on which a mounting ring (4) can be supported, and with a container opening (20) adjoining it, which can be closed with a cover (6), **characterized in,**
**that** in a first mounting step, the mounting ring (4) is guided over the suction bag (3) so that the mounting ring (4) encloses the suction bag (3) and divides it into a lower part (31) closed on one side and an upper part (32) provided with the bag opening (30),
**that** in a second mounting step, the upper part (32) is folded back radially outward over the mounting ring (4) and part of the lower part (31) so that the mounting ring (4) delimits a receiving opening (300) of the suction bag (3),
**that** in a third mounting step, the suction bag (3) is inserted into the container space (200) of the opened container (2) and the mounting ring (4) is supported on the at least one support element (22), and
**that** in a fourth assembly step, the cover (6) or a connection ring (5) connected to the cover (6) is placed on the container (2).

11. Method according to claim 10, **characterized in, that** the mounting ring (4) is pressed tightly against the at least one support element (22) by the cover (6) or by the connection ring (5), which is connected or will be connected to the cover (6).

12. Method according to claim 10 or 11, **characterized in, that** the device (1) is connected to a suction device (8) by a connection line (82) connected to the cover (6), that the suction device (8) is put into operation, and that fluid is transferred to the suction bag (3) via a suction line (81) connected to the cover (6), liquid is transferred into the suction bag (3), after which the cover (6) or the connection ring (5) connected to the cover (6) is detached from the container and the mounting ring (4) is grasped and removed from the container (2) together with the suction bag (3).

13. Method according to claim 12, **characterized in, that** the suction bag (3) comprises a pull cord (34) which is actuated after the suction bag (3) has been removed from the container (2) in order to close the bag opening (30).

14. Method according to claim 12 or 13, **characterized in, that** the mounting ring (4) is removed from the filled suction bag (3) and connected to a new suction bag (3) and the filled suction bag (3) is disposed of, or that the mounting ring (4) is disposed of together with the filled suction bag (3).

## Revendications

1. Dispositif (1) pour un dispositif de succion (8)
avec un conteneur (2), qui présente une paroi du conteneur (24) comportant une face intérieure (241) et une face extérieure (242), qui délimite un espace conteneur (200), et qui est reliée à un fond du conteneur (25) sur la face inférieure et à une ouverture du conteneur (20) sur la face supérieure,
avec une couverture (6) pour fermer l'ouverture du conteneur (20),
avec un anneau de montage (4), et
avec un sac d'aspiration (3) prévu pour un usage unique, qui présente à une extrémité une ouverture du sac (30) et qui est maintenu à l'intérieur de l'espace conteneur (200) par l'anneau de montage (4), qui est divisé en une partie inférieure (31) fermée d'un côté et une partie supérieure (32) pourvue de l'ouverture du sac (30), laquelle partie supérieure (32) est repliée sur l'anneau de montage (4) et une partie de la partie inférieure (31), de sorte que l'anneau de montage (4) délimite une ouverture de réception (300) du sac d'aspiration (3), **caractérisé en ce,**
**qu'**au moins un élément de support (22) est formé sur la face intérieure (241) de la paroi du conteneur (24), sur lequel l'anneau de montage (4) est supporté de telle sorte que la partie supérieure (32) du sac d'aspiration (3) s'étend à l'intérieur de l'espace conteneur (200) entre l'anneau de montage (4) et la paroi du conteneur (24) vers le fond du conteneur (25).

2. Dispositif (1) selon la revendication 1, **caractérisé en ce, que** l'anneau de montage (4) est élastiquement déformable et que l'anneau de montage (4) est fabriqué dans un matériau élastique dur ou élastique souple, de sorte que la partie supérieure (32) du sac d'aspiration (3) peut être de préférence coincée entre l'anneau de montage (4) et la paroi du conteneur (24).

3. Dispositif (1) selon la revendication 1 ou 2, **caractérisé en ce,**
a) **que** ledit au moins un élément de support (22) est formé sous la forme d'un rebord ou d'un épaulement circonférentiel sur la face intérieure de la paroi du conteneur (24); ou
b) **que** ledit au moins un élément de support (22) est une rainure périphérique prévue dans la paroi du conteneur (24) et dont la face inférieure forme de préférence un épaulement; ou
c) **que** ledit au moins un élément de support (22) est un élément annulaire maintenu dans une rainure de la paroi du conteneur (24); ou
d) **que** des éléments de support (22) sont prévus sous forme de moulures dans la paroi du conteneur (24).

4. Dispositif (1) selon une des revendications 1 - 3, **caractérisé en ce,**
a) **que** l'anneau de montage (4) est relié ou peut être relié par complémentarité de forme ou par adhérence à la face intérieure de la paroi du conteneur (24) et est de préférence muni d'au moins un élément de saisie (45); ou
b) **que** l'anneau de montage (4) est maintenu en position par la couverture (6) ou par un anneau de raccordement (5) relié à la couverture (6) ou, de préférence, pressé de manière étanche contre ledit au moins un élément de support (22).

5. Dispositif (1) selon une des revendications 1 - 4, **caractérisé en ce, que** la couverture (6) ou l'anneau de raccordement (5) relié à la couverture (6) comporte une bride annulaire (53) au moyen de laquelle une bague d'étanchéité (29) est pressée de manière étanche contre un bord supérieur ou une face frontale (28) de la paroi du conteneur (24) s'étendant de manière annulaire.

6. Dispositif (1) selon une des revendications 1 - 5, **caractérisé en ce, que** le sac d'aspiration (3) comprend une corde de traction (34) dont l'actionnement permet de fermer le sac d'aspiration (3) ou l'ouverture du sac (30), la corde de traction (34) étant de préférence maintenue dans des œillets ou dans une pièce finale tubulaire (33) du sac d'aspiration (3) qui délimite l'ouverture du sac (30).

7. Dispositif (1) selon une des revendications 1 - 6, **caractérisé en ce, qu'**entre l'ouverture du conteneur (20) et au moins un élément de support (22) est prévu un filetage intérieur (21) sur lequel est vissée la couverture (6) ou l'anneau de raccordement (5) vissé à la couverture (6).

8. Dispositif (1) selon la revendication 7, **caractérisé en ce, que** l'anneau de raccordement (5) comporte, d'un côté, une première pièce filetée (51) vissée au conteneur (5) et, de l'autre côté, une deuxième pièce filetée (52) vissée à la couverture (6) et une bride annulaire (53) située à l'extérieur, à travers laquelle la bague d'étanchéité (29) est pressée de manière étanche contre le bord supérieur (28) du conteneur (2).

9. Dispositif (1) selon une des revendications 1 - 8, **caractérisé en ce, que** la couverture (6) est reliée par des éléments de raccordement (71, 72) à une conduite de raccordement (82) qui est raccordée au dispositif de succion (8) et à une conduite d'aspiration (81).

10. Procédé non chirurgical et non thérapeutique pour monter un sac d'aspiration (3) à usage unique, qui présente à une extrémité une ouverture du sac (30), dans un conteneur (2) d'un dispositif (1) selon l'une des revendications 1 à 9, lequel conteneur (2) comporte un espace conteneur (200) avec au moins un élément de support (22) prévu à l'intérieur, sur lequel peut reposer un anneau de montage (4), et avec une ouverture du conteneur (20) qui s'y raccorde et qui peut être fermée par un couvercle (6), **caractérisé en ce,**
**que**, dans une première étape de montage, l'anneau de montage (4) est guidé par-dessus le sac d'aspiration (3) de manière à ce que l'anneau de montage (4) entoure le sac d'aspiration (3) et le divise en une partie inférieure (31) fermée d'un côté et une partie supérieure (32) pourvue de l'ouverture du sac (30),
**que**, dans une deuxième étape de montage, la partie supérieure (32) est repliée radialement vers l'extérieur sur l'anneau de montage (4) et une partie de la partie inférieure (31), de sorte que l'anneau de montage (4) délimite une ouverture de réception (300) du sac d'aspiration (3),
**que**, dans une troisième étape de montage, le sac d'aspiration (3) est introduit dans l'espace conteneur (200) du conteneur ouvert (2) et que l'anneau de montage (4) est reposé sur le au moins un élément de support (22), et
**que**, dans une quatrième étape de montage, la couverture (6) ou un anneau de raccordement (5) relié à la couverture (6) est placé sur le conteneur (2).

11. Procédé selon la revendication 10, **caractérisé en ce, que** l'anneau de montage (4) est pressé de manière étanche contre au moins un élément de support (22) par la couverture (6) ou par l'anneau de raccordement (5) qui est relié ou sera relié à la couverture (6).

12. Procédé selon la revendication 10 ou 11, **caractérisé en ce, que** le dispositif (1) est relié à un dispositif de succion (8) par une conduite de raccordement (82) raccordée à la couverture (6), que le dispositif de succion (8) est mis en service et que le liquide est transféré dans le sac d'aspiration (3) via une conduite d'aspiration (81) reliée à la couverture (6), après quoi la couverture (6) ou l'anneau de raccordement (5) relié à la couverture (6) est détaché du conteneur et l'anneau de montage (4) est saisi et retiré du conteneur (2) avec le sac d'aspiration (3).

13. Procédé selon la revendication 12, **caractérisé en ce, que** le sac d'aspiration (3) comprend une corde de traction (34) qui est actionnée après le retrait du sac d'aspiration (3) du conteneur (2) afin de fermer l'ouverture du sac (30).

14. Procédé selon la revendication 12 ou 13, **caractérisé en ce, que** l'anneau de montage (4) soit retiré du sac d'aspiration (3) rempli et relié à un nouveau sac d'aspiration (3) et que le sac d'aspiration (3) rempli soit éliminé ou que l'anneau de montage (4) soit éliminé avec le sac d'aspiration (3) rempli.
